# EUROPEAN PATENT SPECIFICATION

(11) **EP 2 415 477 B1**
(45) Date of publication and mention of the grant of the patent: **30.04.2014**
(21) Application number: 10757989.8
(22) Date of filing: 04.03.2010
(51) Int. Cl.: A61K 36/30, A61K 35/64, A61K 35/12, A61K 9/06, A61P 17/02, A61P 31/04, A61K 47/44

(54) **TRADITIONAL CHINESE MEDICINAL OINTMENT FOR TREATING BURN, SCALD AND INFECTIVE TRAUMA AND PREPARATIVE METHOD THEREOF**
SALBE NACH ART DER TRADITIONELLEN CHINESISCHEN MEDIZIN ZUR BEHANDLUNG VON VERBRENNUNGEN, VERBRÜHUNGEN, INFEKTIONSTRAUMATA UND HERSTELLUNGSVERFAHREN DAFÜR
POMMADE DE MÉDECINE CHINOISE TRADITIONNELLE POUR TRAITER LES BRÛLURES, LES ÉCHAUDURES ET LES TRAUMATISMES INFECTIEUX ET PROCÉDÉ DE PRÉPARATION DE CELLE-CI

(30) Priority: 02.04.2009 CN 200910020093
(43) Date of publication of application: 08.02.2012
(73) Proprietor: Sun, Xinwei, Jinan, Shandong 250100 (CN)
(72) Inventor: Sun, Xinwei, Jinan, Shandong 250100 (CN)
(74) Representative: Decamps, Alain René François
(86) International application number: PCT/CN2010/000259
(87) International publication number: WO 2010/111886

(56) References cited:
- WO-A2-97/42963
- CN-A- 1 218 672
- CN-A- 101 513 441
- DATABASE WPI Week 200925 Thomson Scientific, London, GB; AN 2009-F45823 XP002684860, & CN 101 361 842 A (LI C) 11 February 2009 (2009-02-11)
- DATABASE WPI Week 200156 Thomson Scientific, London, GB; AN 2001-503330 XP002684861, & CN 1 298 725 A (GAO J) 13 June 2001 (2001-06-13)
- DATABASE BIOSIS [Online] BIOSCIENCES INFORMATION SERVICE, PHILADELPHIA, PA, US; December 2008 (2008-12), PAPAGEORGIOU V P ET AL: "Alkannins and Shikonins: A New Class of Wound Healing Agents", XP002684862, Database accession no. PREV200900074158 & CURRENT MEDICINAL CHEMISTRY, vol. 15, no. 30, December 2008 (2008-12), pages 3248-3267, ISSN: 0929-8673
- OZGEN U ET AL: "Fibroblast growth stimulation by extracts and compounds of Onosma argentatum roots", JOURNAL OF ETHNOPHARMACOLOGY, ELSEVIER SCIENTIFIC PUBLISHERS LTD, IE, vol. 104, no. 1-2, 8 March 2006 (2006-03-08), pages 100-103, XP027939413, ISSN: 0378-8741 [retrieved on 2006-03-08]
- XIN CHEN ET AL: "Shikonin, a component of antiinflammatory Chinese herbal medicine, selectively blocks chemokine binding to CC chemokine receptor-1", INTERNATIONAL IMMUNOPHARMACOLOGY, vol. 1, no. 2, 1 February 2001 (2001-02-01), pages 229-236, XP55040386, ISSN: 1567-5769, DOI: 10.1016/S1567-5769(00)00033-3
- HE JIMING: 'The observation of therapeutic effect on 28 burn cases treated by ZICAO ointment' JOURNAL OF HUNAN COLLEGE OF TRADITIONAL CHINESE MEDICINE vol. 9, no. 4, 1989, pages 190 - 191

## Description

### Technical field

The present invention relates to an ointment based on traditional Chinese medicine for the treatment of burn, scald and/or infectious trauma and its preparation.

### Background technology

Burns or scalds are extremely painful for patients and current treatments are expensive. Even with such costly treatment, a complete recovery is hardly obtained and these patients are often left with scars. Moreover, treating the scars while the wound is healing is extremely painful for these patients. Regarding infectious trauma, such as wound discharging pus, a debridement is required during the treatment, which is painful, and generally left the patients with scars.

### Contents of the invention

The present invention relates to a new ointment based on traditional Chinese medicine for the treatment of burn, scald and/or infectious trauma and its preparation. Said invention which has a low cost, favorable therapeutic effects, a short time curing and does not leave scars after treatment, solves the problems exposed including high cost of drugs for burn, scald and/or infectious trauma, scars left after treatment and unfavorable therapeutic effects.

For this purpose, said invention discloses:

An ointment based on traditional Chinese medicine for the treatment of burn, scald and/or infectious trauma, which comprises, in weight of every 500 portions, the following ingredients: 4-10 portions of Radix Arnebiae seu Lithospermi, 5-20 potions of Cera Flava, 5-20 portions of Cera Chinensis, and 450-486 portions of lard.

Advantageously, the ointment comprises, in weight of every 500 portions, the following ingredients: 6 portions of Radix Arnebiae seu Lithospermi, 8 potions of Cera Flava, 10 portions of Cera Chinensis and 476 portions of lard.

Alternatively the ointment can comprises, in weight of every 500 portions, the following ingredients: 8 portions of Radix Arnebiae seu Lithospermi, 10 potions of Cera Flava, 15 portions of Cera Chinensis and 467 portions of lard.

The method for the preparation of said ointment based on traditional Chinese medicine for the treatment of burn, scald and/or infectious trauma is disclosed as follows: after said lard is heated to 200-230 °C, said portions of Radix Arnebiae seu Lithospermi, Cera Flava and Cera Chinensis are added respectively, the mixture is then fully stirred until melting of said Cera Flava and Cera Chinensis, the heating is then stopped and the mixture is filtrated while still hot to remove the herbal residues and let the filtrate to cool down at room temperature.

Although said invention may just contain four ingredients, each has a significant therapeutic effect, due to their specific compatibility and defined doses. The ingredients are detailed below.

Radix Arnebiae seu Lithospermi, which has bitter and cold properties, belongs to the heart and liver meridian and is mainly used to cool and activate blood, detoxify toxins, promote eruption and vesiculation. In addition, it can also treat hematemesis; hemorrhinia, hematuria, purpura, macule, measles, jaundice, carbuncle abscess, and burn (ref. Dictionary of Traditional Chinese Medicine, 2nd edition, pp. 3270-3273).

Cera Flava, has sweat, light, and smooth properties, belongs to spleen, stomach and large intestine meridian, and is mainly used to detoxify toxin, promote granulation and stop bleeding. Besides, it can also treat carbuncle abscess, ulcer, dysentery and metrostaxis of fetal movement (ref. Dictionary of Traditional Chinese Medicine, 2nd edition, pp. 3486-3487).

Cera Chinensis, has sweat, light, and warm properties, belongs to liver meridian, and is mainly used to stop bleeding, promote granulation and control pain. Moreover, it can also treat hemorrhage or incised wound, hematuria, hematochezia, and healing difficulty of ulcers (ref. Dictionary of Traditional Chinese Medicine, 2nd edition, pp. 1214-1215).

Lard, as sweat and slightly cold properties, is mainly used to reinforce deficiency, moisturize dryness, and detoxify toxins. It can also treat consumptive diseases, constipation, skin chap, cough due to lung deficiency, malignant score, and scalds and burns (ref. Dictionary of Traditional Chinese Medicine, 2nd edition, pp. 3073-3074).

Properly adjusting the proportions of each ingredient not only reduces the drug cost, but also ensures its efficiency. Furthermore, the respective property of each drug is fully considered during the formulation, thus optimizing the therapeutic effects.

Application: Smear the drug evenly on sterile medicinal cotton which covers the wound. If the wound is too large, several medicinal cotton should be used to evenly cover the whole wound, then bind up and fix said medicinal cotton with gauze. Once applied, the patient may dress and cover the quilt. The bind up with gauze must not be too tight and the gauze should not be cover by an air impermeable material, such as plastic tissue, for the binding up. Based on the wound significance and the drug absorption, the dressing is changed every 24, 12 or 6 hours.

Effects: The drug is a red semisolid ointment, which has antibacterial and anti-inflammatory effect, activates and cools blood, eliminates swelling, reduces pain, and eliminates the necrotic tissue, promotes granulation and facilitates skin growth, thus makes it an ideal drug for burn, scald and/or infectious trauma.

### Properties of the drug

1. The period between the application and the effect is short. 2-3 minutes after the application, the drug effectively controls the pain.
2. Favorable permeability: The drug diffuses rapidly into the wound promoting tissue restoration, liquefying and discharging necrotic tissue.
3. Potent anti-infection effect: The drug has antibacterial and anti-inflammatory effects, does not require sterile conditions, and has favorable effect on infected wounds with pus.
4. Soften wounds and keep moisture: The drug can soften dry, hard crusted wounds and remove the crust after it fully permeates the wounds, maintaining the wounds moist and promoting the wounds healing.
5. Accelerated wound healing: The drug forms a protective layer on the wound, which is helpful for wound healing, and does not leave scars after complete healing.
6. Convenient to use: After Smearing the drug in sterile medicinal cotton which covers the wound. The patient can dress and cover quilt, which means exposure therapy is unnecessary.

### Examples of treatment

This drug has been applied to hundreds of patients, with significant efficacy. Some cases are listed below.

### I. Burns

An old woman in her sixties presented to a hospital with burns in hands, feet and legs caused by the outbreak of a fire of a gas burner. During several days in hospital, she had fever, and used antibiotics through persistent intravenous drip, but with limited effects. She was introduced to our hospital. At admission, the wound was black with dry, hard crust, below which there was pus. The wounded limbs were swollen and sore. The drug was applied, and the dressing was changed every 24 hours. Crusts (3-4 millimeters thick) were soften and fell off the wound gradually, and totally fell off 3 days later. At that time, the wound with substantial pus was exposed. Subsequently, the wound was cleaned, and the treatment was pursued for 2 weeks. At the end of the treatment, the wound was completely healed and the skin was red and smooth without scar.

A man in his forties whose chest and hands were burned by gasoline was introduced to us. On examination, the epidermis on the wound totally fell off, and the wound was red, swelling and painful. The drug was applied, and the dressing was changed every 12 hours. The wound was cured after 10 days without scar.

### II. Oil scald

A male construction worker in his thirties lost his balance by accident while boiling asphalt. His hands had pressed the asphalt, and were severely scalded. The asphalt was stripped at hospital. After more than 10 days of treatment, the effects were not obvious. He was introduced to us. The drug was applied, and dressing was changed every 12 hours. The wound recovered after 2 weeks without scar.

A woman in her forties, whose face, chest and arms were scalded by hot oil during frying dough sticks, came to us. The epidermis on the wound partly fell off, and the wound was red, swollen and painful. This drug was applied, and the dressing was changed every 12 hours. The wound recovered after 10 days without scar.

### III. Scalded by boiled water

A one-year old little boy was scalded by boiled water. He was treated a week for an ulcer. The hospital suggested an amputation, which was refused by his parents. He was introduced to us. His pain was controlled immediately after using our drug, and he did not cried. The dressing was changed every 12 hours. The wound recovered after a dozen of days without scar.

A five year old girl knocked over a tub with boiled water by accident. The water poured to her head, causing her face swollen. The epidermis fell off, and she felt intense pain. She was sent to us immediately. The drug was applied, and she did not cry any more. The dressing was changed every 12 hours. The wound recovered after one week without scar.

### IV. Infectious trauma

A woman in her fifties scratched her instep with a grapple by accident while doing farm work and had a penetrating wound. Later, the wound was infected and discharged pus, with swelling and aggravating ulcer. She received antibiotics infusion in a country clinic for more than 10 days, but with limited efficacy. So she came to us. The drug was applied, and the dressing was changed every 12 hours. After one week, her swelling and pus resolved and 4-5 days later, she completely recovered without scar.

An old man in his seventies developed ischemic necrotic suppuration at his buttock, and erosive ulcer exposing his coccyx, due to a long stay in a hospital bed. The therapeutic effect was unfavorable, and the ulcer did not healed despite a long period of treatment. His son brought him to us. The drug was applied, and the dressing was changed every 24 hours. The wound healed after more than one month without scar.

### Specific mode of execution

The invention is further described with following examples:

### Example 1:

An ointment based on traditional Chinese medicine for the treatment of burn, scald and/or infectious trauma, comprising, in weight of every 500 portions, the following ingredients: 4 portions of Radix Arnebiae seu Lithospermi , 5 potions of Cera Flava, 5 portions of Cera Chinensis and 486 portions of lard.

The ointment is prepared as follows: after said lard is heated to 200 °C, said portions of Radix Arnebiae seu Lithospermi, Cera Flava and Cera Chinensis are added, respectively, the mixture is then fully stirred until melting of said Cera Flava and Cera Chinensis, the heating is then stopped and the mixture is filtrated to remove the herbal residues while still hot, then the filtrate is cooled down at room temperature.

### Example 2:

An ointment based on traditional Chinese medicine for the treatment of burn, scald and/or infectious trauma, comprising, in weight of every 500 portions, the following ingredients: 6 portions of Radix Arnebiae seu Lithospermi , 8 potions of Cera Flava, 10 portions of Cera Chinensis and 476 portions of lard.

The ointment is prepared as follows: after said lard is heated to 210 °C, said portions of Radix Arnebiae seu Lithospermi, Cera Flava and Cera Chinensis are added, respectively, the mixture is then fully stirred until melting of said Cera Flava and Cera Chinensis, the heating is then stopped and the mixture is filtrated to remove the herbal residues while still hot, then the filtrate is cooled down at room temperature.

### Example 3:

An ointment based on traditional Chinese medicine for the treatment of burn, scald and/or infectious trauma, comprising, in weight of every 500 portions, the following ingredients: 8 portions of Radix Arnebiae seu Lithospermi, 10 potions of Cera Flava, 15 portions of Cera Chinensis and 467 portions lard.

The ointment is prepared as follows: after said lard is heated to 220 °C, said portions of Radix Arnebiae seu Lithospermi, Cera Flava and Cera Chinensis are added, respectively, the mixture is then fully stirred until melting of said Cera Flava and Cera Chinensis, the heating is then stopped, and the mixture is filtrated to remove the herbal residues while still hot, then the filtrate is cooled down at room temperature.

### Example 4:

An ointment based on traditional Chinese medicine for the treatment of burn, scald and/or infectious trauma comprising, in weight of every 500 portions, the following ingredients: 10 portions of Radix Arnebiae seu Lithospermi, 20 potions of Cera Flava, 20 portions of Cera Chinensis and 467 portions of lard.

The ointment is prepared as follows: after lard is heated to 230 °C, said portions of Radix Arnebiae seu Lithospermi, Cera Flava and Cera Chinensis are added, respectively, the mixture is then fully stirred until melting of said Cera Flava and Cera Chinensis, the heating is then stopped and the mixture is filtrated to remove the herbal residues while still hot, then the filtrate is cooled down at room temperature.

## Claims

1. Ointment based on traditional Chinese medicine comprising, in weight of every 500 portions, the following ingredients: 4-10 portions of Radix Arnebiae seu Lithospermi, 5-20 potions of Cera Flava, 5-20 portions of Cera Chinensis and 450-486 portions of lard for use as a medicament.

2. Ointment based on traditional Chinese medicine for use in the treatment of burn, scald and/or infectious trauma, said ointment comprising, in weight of every 500 portions, the following ingredients: 4-10 portions of Radix Arnebiae seu Lithospermi, 5-20 portions of Cera Flava, 5-20 portions of Cera Chinensis and 450-486 portions of lard.

3. Ointment according to claim 1 or 2, comprising, in weight of every 500 portions, the following ingredients: 6 portions of Radix Arnebiae seu Lithospermi, 8 portions of Cera Flava, 10 portions of Cera Chinensis and 476 portions of lard.

4. Ointment according to claim 1 or 2, comprising, in weight of every 500 portions, the following ingredients: 8 portions of Radix Arnebiae seu Lithospermi, 10 portions of Cera Flava, 15 portions of Cera Chinensis and 467 portions of lard.

5. Method for the preparation of an ointment based on traditional Chinese medicine as described in any of the claims 1 to 4, **characterized in that** after said lard is heated to 200-230 °C, said portions of Radix Arnebiae seu Lithospermi, Cera Flava and Cera Chinensis are added, respectively, the mixture is then fully stirred until melting of said Cera Flava and Cera Chinensis, said heating is then stopped and the mixture is filtrated while still hot to remove the herbal residues and the filtrate is let to cool down at room temperature.

## Patentansprüche

1. Salbe auf der Grundlage der traditionellen chinesischen Medizin, umfassend, in Gewicht von jeweils 500 Portionen, die Folgenden Inhaltsstoffe: 4 - 10 Portionen Radix Arnebiae seu Lithospermi, 5 - 20 Portionen Cera Flava, 5 - 20 Portionen Cera Chinensis und 450 - 486 Portionen Schmalz zur Verwendung als ein Arzneimittel.

2. Salbe auf der Grundlage der traditionellen chinesischen Medizin zur Verwendung bei der Behandlung von Verbrennungen, Verbrühungen und/oder Infektionstraumata, wobei die Salbe, in Gewicht von jeweils 500 Portionen, die Folgenden Inhaltsstoffe umfasst: 4 - 10 Portionen Radix Arnebiae seu Lithospermi, 5 - 20 Portionen Cera Flava, 5 - 20 Portionen Cera Chinensis und 450 - 486 Portionen Schmalz.

3. Salbe nach Anspruch 1 oder 2, umfassend, in Gewicht von jeweils 500 Portionen, die Folgenden Inhaltsstoffe: 6 Portionen Radix Arnebiae seu Lithospermi, 8 Portionen Cera Flava, 10 Portionen Cera Chinensis und 476 Portionen Schmalz.

4. Salbe nach Anspruch 1 oder 2, umfassend, in Gewicht von jeweils 500 Portionen, die Folgenden Inhaltsstoffe: 8 Portionen Radix Arnebiae seu Lithospermi, 10 Portionen Cera Flava, 15 Portionen Cera Chinensis und 467 Portionen Schmalz.

5. Verfahren zur Zubereitung einer Salbe auf der Grundlage der traditionellen chinesischen Medizin, wie in einem der Ansprüche 1 bis 4 beschrieben, **dadurch gekennzeichnet, dass**, nachdem das Schmalz auf 200 - 230 °C erhitzt wurde, die Portionen Radix Arnebiae seu Lithospermi, Cera Flava und Cera Chinensis jeweils hinzugefügt werden, die Mischung dann vollständig gerührt wird, bis das Cera Flava und das Cera Chinensis schmelzen, die Erhitzung dann gestoppt wird und die Mischung gefiltert wird, während sie noch heiß ist, um die pflanzlichen Rückstände zu entfernen, und das Filtrat auf Raumtemperatur abkühlen gelassen wird.

## Revendications

1. Pommade de médecine chinoise traditionnelle comprenant, en poids pour respectivement 500 parties, les ingrédients suivants : 4 à 10 parties de Radix Arnebiae seu Lithospermi, 5 à 20 parties de Cera Flava, 5 à 20 parties de Cera Chinensis et 450 à 486 parties de saindoux pour son utilisation comme médicament.

2. Pommade de médecine chinoise traditionnelle, pour son utilisation dans le traitement des brûlures, des échaudures et/ou des traumatismes infectieux, ladite pommade comprenant, en poids pour respectivement 500 parties, les ingrédients suivants : 4 à 10 parties de Radix Arnebiae seu Lithospermi, 5 à 20 parties de Cera Flava, 5 à 20 parties de Cera Chinensis et 450 à 486 parties de saindoux.

3. Pommade selon la revendication 1 ou 2, comprenant en poids pour respectivement 500 parties, les ingrédients suivants : 6 parties de Radix Arnebiae seu Lithospermi, 8 parties de Cera Flava, 10 parties de Cera Chinensis et 476 parties de saindoux.

4. Pommade selon la revendication 1 ou 2, comprenant en poids pour respectivement 500 parties, les ingrédients suivants : 8 parties de Radix Arnebiae seu Lithospermi, 10 parties de Cera Flava, 15 parties de Cera Chinensis et 467 parties de saindoux.

5. Procédé pour la préparation d'une pommade de médecine chinoise traditionnelle décrite selon l'une quelconque des revendications 1 à 4, **caractérisé en ce que**, après que ledit saindoux est chauffé à 200-230°C, lesdites parties de Radix Arnebiae seu Lithospermi, de Cera Flava et de Cera Chinensis sont ajoutées, respectivement, le mélange est ensuite complètement agité jusqu'à la fusion desdits Cera Flava et de Cera Chinensis, ledit chauffage est ensuite stoppé et le mélange est filtré alors qu'il est encore chaud pour éliminer les résidus herbacés et on laisse refroidir le filtrat à température ambiante.
